# EUROPÄISCHE PATENTSCHRIFT

(11) **EP 2 407 085 B1**
(45) Veröffentlichungstag und Bekanntmachung des Hinweises auf die Patenterteilung: **11.09.2013**
(21) Anmeldenummer: 11174091.6
(22) Anmeldetag: 15.07.2011
(51) Int. Cl.: A61B 1/00, A61B 1/05, H01B 11/00

(54) **Endoskopisches Instrument und Verwendung eines Sternviererkabels**
Endoscopic instrument and use of a star quad cable
Instrument d'endoscopie et utilisation d'un câble starquad

(30) Priorität: 15.07.2010 DE 102010027400
(43) Veröffentlichungstag der Anmeldung: 18.01.2012
(73) Patentinhaber: Karl Storz GmbH & Co. KG, 78532 Tuttlingen (DE)
(72) Erfinder: Baum, Eckhart, 78589 Dürbheim (DE); Schwarz, Peter, 78532 Tuttlingen-Nendingen (DE)
(74) Vertreter: Witte, Weller & Partner

(56) Entgegenhaltungen:
- JP-A- 2005 160 925
- US-A1- 2004 197 058
- US-A1- 2005 029 006
- US-A1- 2010 261 961

## Beschreibung

Die vorliegende Erfindung betrifft ein endoskopisches Instrument, insbesondere ein endoskopisches medizinisches Instrument, mit einem Schaft, der ein distales Ende, an dem mindestens ein optischer Sensor, insbesondere ein optoelektronischer Bildsensor, angeordnet ist, und ein proximales Ende zum Anschluss an eine Versorgungseinheit aufweist, und mit einem Datenübertragungselement, das zwischen dem mindestens einen optischen Sensor und dem proximalen Ende angeordnet ist und dafür ausgebildet ist, mindestens zwei Signale differentiell zu übertragen.

Derartige endoskopische Instrumente sind bekannt und werden bspw. von der Firma Karl Storz GmbH & Co. KG unter Einsatz von Stablinsen-Endoskopen in Verbindung mit der Image 1® HD Endokamera vertrieben.

Grundsätzlich ist es bekannt und seit langer Zeit üblich, endoskopische optische bzw. optoelektronische Sensoren aus CCD (Charge-Coupled Device)- oder CMOS (Complementary Metal Oxide Semiconductor)-Modulen aufzubauen. Die Anforderungen an geringe Dimensionen des endoskopischen Instruments machen es in der Regel unmöglich, Bilddatenverarbeitungs- und Steuerungseinheiten dieser Sensoren an dem distalen Ende des endoskopischen Instruments vorzusehen. Daher sind Datenübertragungselemente angeordnet, die die Bilddaten von den optischen Sensoren zu einer Versorgungseinheit übertragen und umgekehrt Steuerungssignale bzw. Ansteuertakte von der Versorgungseinheit an den optischen Sensor übermitteln.

Zur Datenübertragung wurden bei analogen Videosignalen und relativ hochfrequenten Ansteuertakten in der Regel Koaxialleiter von mehreren Metern Länge verwendet. Die Verwendung von Koaxialleitern ist bei Frequenzen von mehr als einem Megahertz (MHz) üblich, da sie eine geringe Signaldämpfung aufweisen und im Allgemeinen bei hohen Frequenzen nebensprechfrei sind. Die Datenübertragungssysteme für bekannte endoskopische optische Sensoren, bspw. Kamera- oder Videomodule, die die Bilddaten in einem PAL- oder NTSC-Standardformat übertraten, weisen meist mehrere Koaxialleiter für die Bilddaten und hochfrequenten Ansteuertakte und weitere Einzelelemente für die Spannungsversorgung, ein Shuttersignal oder niederfrequente Ansteuertakte auf.

Beispiele für Endoskopsysteme mit Koaxialleitern finden sich bspw. in den Druckschriften DE 197 35 989 A1 und DE 38 17 915 C2.

Im Bereich der endoskopischen optischen Sensoren haben sich die Anforderungen an die Bildqualität und Bildauflösung in der letzten Zeit ständig erhöht. Entsprechend steigt damit auch die geforderte Bandbreite der notwendigen digitalen Datenübertragung. Parallel-Schnittstellen sind dabei schwierig zu realisieren, da sie viele Verbindungen erfordern und einen hohen Leistungsverbrauch aufweisen. Platzbedarf und Leistungsverbrauch sind jedoch regelmäßig Parameter, die in endoskopischen Anwendungen kritisch sind und niedrig gehalten werden sollten. Daher werden serielle Breitbandschnittstellen zur analogen und digitalen Datenübertragung gefordert.

Es wurde daher bei neueren endoskopischen optischen Sensoren vorgeschlagen, das Bilddatensignal des optischen Sensors auf ein differentielles serielles LVDS (Low Voltage Differential Signalling)-Signal zu wandeln, um diesen Schnittstellen-Standard zur Hochgeschwindigkeits-Bilddatenübertragung zu verwenden. Die Bilddatenwandlung erfordert jedoch ein zusätzliches Wandlerelement, das wiederum Platzbedarf hat. Als Datenübertragungselemente werden dabei so genannte Twisted-Pair-Kabel verwendet. Diese Twisted-Pair-Kabel sind Kabel mit verdrillten Aderpaaren. Die verdrillten Aderpaare werden mit symmetrischen Signalen beaufschlagt, um am proximalen Ende des Datenübertragungselements die Differenz zwischen den Signalen der beiden Adern bspw. mittels eines Differenzverstärkers auszuwerten. Auf diese Weise ist es in der Regel möglich, das am distalen Ende beaufschlagte Signal optimal auf der proximalen Empfängerseite zu rekonstruieren.

Beispiele für Twisted-Pair-Kabel finden sich bspw. in den Druckschriften EP 0 819 311 B1 und EP 0 946 951 B1.

Diese Twisted-Pair-Kabel weisen jedoch aufgrund der Verdrillung der Adern einen relativ großen Durchmesser auf, um bei der geforderten Kabellänge und Übertragungsbandbreite eine ausreichend geringe Signaldämpfung zu besitzen. Dies würde jedoch auch zwangsläufig den Durchmesser des endoskopischen Instruments erhöhen, wobei der Einsatz am Menschen jedoch möglichst geringe Durchmesser fordert.

Hochauflösende optische Sensoren werden zunehmend vor allem auch im Telekommunikationsbereich in großen Stückzahlen für Endverbraucher angeboten. Die Verwendung erfolgt bspw. in Mobiltelefonen oder digitalen Fotokameras. Diese optischen Sensoren weisen zunehmend den so genannten MIPI CSI-2 (Camera Serial Interface)-Standard auf. Ein Beispiel hierfür findet sich in der Druckschrift DE 10 2007 038725 B3.

Der MIPI CSI-2-Standard definiert ein serielles, schnelles und kosteneffizientes Interface zwischen einem peripheren Bildmodul für mobile tragbare Geräte mit geringen Dimensionen. Es handelt sich im Speziellen um ein serielles Breitbandinterface zur Übertragung von Bild- und Videodaten über kurze Strecken von weniger als 30 cm innerhalb mobiler Anwendergeräte.

Die Verwendung von miniaturisierten CMOS-Sensoren, die in großen Stückzahlen für den Telekommunikationsbereich entwickelt und angeboten werden, ist grundsätzlich auch im Bereich der endoskopisch Instrumente interessant. In diesem Bereich wird bisher oft auf Eigenentwicklungen zurückgegriffen, die entsprechend kostenaufwändig sind. Bei der Verwendung der aus dem Telekommunikationsbereich bekannten Bildsensoren müsste jedoch ebenfalls das entsprechende MIPI CSI-2-Interface verwendet werden. Zu dieser Datenübertragung werden im Telekommunikationsbereich flexible Leiterplatten oder die voranstehend beschriebenen Twisted-Pair-Leitungen verwendet, was aufgrund der kurzen Kabellängen in Mobilfunkgeräten bei der gegebenen Dämpfung problemlos möglich ist.

In endoskopischen Anwendungen sind jedoch in der Regel deutlich längere Übertragungswege zu überbrücken, so dass zur Beibehaltung einer geringen Dämpfung entsprechend große Kabeldurchmesser zu wählen wären, was bisher die Anwendung in endoskopischen Bereichen ausgeschlossen hat. Eine Lösung dieses Problems hätte für endoskopische Anwendungen große wirtschaftliche Vorteile und könnte die Bildübertragungsqualität maßgeblich erhöhen.

US 2010/0261961 A1 zeigt ein endoskopisches Instrument, wie es eingangs genannt wurde.

JP 2005-160925 zeigt ebenfalls ein eingangs genanntes Endoskop, bei dem ein Twisted-Pair-Kabel verwendet wird, um den Durchmesser gegenüber einem herkömmlichen Koaxialkabel zu reduzieren.

US 2005/0029006 A1 zeigt im Hinblick auf den IEEE 1394-Standard, dass in bestimmten Fällen ein Twisted-Pair-Kabel durch ein Sternviererkabel ersetzt werden kann.

Es ist daher eine Aufgabe der vorliegenden Erfindung, eine Möglichkeit aufzuzeigen, die die aus dem Telekommunikationsbereich bekannten Bildsensoren und digitalen Signalschnittstellen auch auf dem Gebiet der Endoskopie unter Beachtung der Dimensionen der Instrumente und Kabeldurchmesser zur Verfügung stellt.

Es wird daher vorgeschlagen, das eingangs genannte endoskopische Instrument dahingehend weiterzubilden, dass das Datenübertragungselement als Sternviererkabel gemäß Anspruch 1 ausgebildet ist.

Der Einsatz eines Sternviererkabels als Datenübertragungselement ermöglicht es, bspw. CMOS- oder CCD-Sensoren mit HD-Auflösungen von 1920 x 1080 Bildpunkten zu verwenden, da man in der Lage ist, Übertragungsraten von über 400 MBit/s mit geringem Kabeldurchmesser und entsprechend kleinem Bauraum an dem distalen Ende einzusetzen. Das Sternviererkabel stellt einen geringen Kabeldurchmesser bei gleichzeitig geringer Dämpfung bereit. Durch die differentielle Signalübertragung wird eine rauscharme Signalrekonstruktion an dem proximalen Ende bzw. in der Versorgungseinheit ermöglicht, obwohl ein sehr geringer Leitungsquerschnitt eingesetzt wird.

In den vorliegend verwendeten Frequenzbereichen ist die Dämpfung eines Kabels vorwiegend durch den dem Fachmann bekannten Skineffekt dominiert. Die durch den Skineffekt bedingten Verluste steigen mit zunehmender Signalfrequenz (das heißt mit zunehmender Bitrate) und kleiner werdendem Kabeldurchmesser an. Bei hohen Auflösungen mit Bildwiederholungsraten von etwa 30 Bildern pro Sekunde werden Datenraten von mehr als 400 MBit/s, insbesondere bis zu 960 MBit/s, benötigt. Ein Datenübertragungselement, das zur Übertragung solcher Bitraten über eine gewisse Länge, das heißt von dem distalen Ende mindestens zu dem proximalen Ende, geeignet sein soll, muss somit abhängig von seiner Bauart bei Berücksichtigung des Skineffekts einen Mindestdurchmesser aufweisen.

Die Verwendung eines Datenübertragungselements mit einem Sternvierer-Aufbau, insbesondere als symmetrischer Aufbau, löst den bei endoskopischen Anwendungen vorliegenden Konflikt zwischen geringen äußeren Dimensionen und hoher Übertragungsqualität und -bandbreite. Da eine Sternviererkabel-Anordnung durch seinen kompakten Aufbau im Vergleich zu anderen Datenübertragungselementen bei gleichem Durchmesser eine niedrigere Dämpfung aufweist, ist man so in der Lage, auch bei endoskopischen Anwendungen und kleinsten geforderten Durchmessern eine optimale Übertragungsqualität auch bei hohen Auflösungen von 1920 x 1080 Bildpunkten und entsprechender Bildfrequenz (30/60 Hz) bereitzustellen.

Die einzelnen Übertragungspaare bzw. Adernpaare der Sternvierer-Anordnung benötigen zudem keine Abschirmung gegeneinander, da ein eventuelles Übersprechen immer auf beide Adern des anderen Adernpaares gleich einwirkt und somit durch den jeweiligen differentiellen Empfänger aufgehoben wird.

Unter "endoskopischen Instrumenten" sind insbesondere sämtliche denkbaren endoskopischen medizinischen Instrumente zu verstehen, die in den Körper eines Patienten eingeführt werden können und einen optischen Sensor an ihrem Distalende aufweisen. Insbesondere handelt es sich hierbei um ein Endoskop oder einen Katheter. Dies ist jedoch nicht zwingend, es kann sich auch um ein endoskopisches Instrument in einer anderen Anwendung handeln, insbesondere auf Gebieten, in den der Bauraum ein kritischer Parameter ist.

Unter einer "Versorgungseinheit" des endoskopischen Instruments ist allgemein der proximalseitige Geräteaufbau zu verstehen, der zum Betrieb des endoskopischen Instruments dient und notwendig ist. Die Versorgungseinheit kann bspw. die Spannungsversorgung des endoskopischen Instruments zur Verfügung stellen, zum Anschluss von Lichtleitkabeln oder der Versorgung mit Luft und pneumatischem Druck dienen, oder eine eventuell in dem endoskopischen Instrument befindliche Absaugeinrichtung betreiben. Die Versorgungseinheit kann auch Bildverarbeitungseinrichtungen zur Verarbeitung der von dem mindestens einen optischen Sensor gelieferten Bilddaten bereitstellen und Anzeigevorrichtungen zur Darstellung der Bilddaten aufweisen. Die Versorgungseinrichtung kann auch Bedienelemente umfassen, die zum Betrieb des endoskopischen Instruments dienen.

Bei dem optischen Sensor handelt es sich insbesondere um eine Kamera, die für Einzelbild- und/oder Videoaufzeichnungen geeignet ist.

Die Verwendung eines Sternviererkabels zur Datenübertragung zu und/oder von mindestens einem optischen Sensor in einem endoskopischen Instrument stellt gemäß einem zweiten Aspekt der Erfindung dieselben Vorteile wie das voranstehend beschriebene endoskopische Instrument gemäß der vorliegenden Erfindung bereit.

Die eingangs gestellte Aufgabe wird somit vollständig gelöst.

Wenn lediglich zwei differentielle Signale übertragen werden sollen, ist eine Ausgestaltung vorteilhaft, bei der ein erstes Signal ein Bilddatensignal und ein zweites Signal ein Taktsignal ist. Insbesondere werden das erste und das zweite Signal differentiell übertragen.

Bei einer vorteilhaften Ausgestaltung der Erfindung ist der mindestens eine optische Sensor mit dem Sternviererkabel derart verbunden, dass mindestens drei Signale differentiell übertragen werden können.

Hierdurch wird bspw. bereits der Betrieb eines zweiten optischen Sensors möglich. In diesem Fall stehen zwei Datenübertragungskanäle dafür bereit, die Bildinformation der zwei Sensoren getrennt zu übertragen. Ein weiterer Datenkanal steht dafür bereit, ein Taktsignal zu übertragen, das für beide optischen Sensoren dann identisch ist. Auf diese Weise kann bspw. ein distalseitiges Video-Stereosystem umgesetzt werden.

Alternativ kann bei einem Aufbau mit nur einem optischen Sensor auch das Bilddatensignal auf zwei Kanäle aufgeteilt werden. Durch die bei gleicher Auflösung geringere Übertragungsbandbreite pro Kanal können so längere Distanzen bei gleichbleibender Signalqualität überbrückt werden.

Bei einer weiteren vorteilhaften Ausgestaltung der Erfindung ist der mindestens eine optische Sensor mit dem Sternviererkabel derart verbunden, dass vier Signale differentiell übertragen werden können.

Auf diese Weise ist es möglich, zwei getrennte Bilddatensignale und zwei getrennte Taktsignale zu übertragen. Dies ermöglicht insbesondere den Einsatz von zwei optischen Sensoren, die nicht notwendigerweise mit demselben Taktsignal gesteuert werden müssen. Auf diese Weise kann ebenfalls ein Stereosystem bzw. Stereoendoskop verwirklicht werden, aber auch bspw. ein Fluoreszenzendoskop realisiert werden.

Insbesondere können ein erster und ein zweiter optischer Sensor bereitgestellt werden, wobei ein erstes Signal ein Bilddatensignal und ein zweites Signal ein Taktsignal des ersten optischen Sensors ist, und wobei ein drittes Signal ein Bilddatensignal und ein viertes Signal ein Taktsignals des zweiten optischen Sensors ist. Insbesondere werden das erste, das zweite, das dritte und das vierte Signal differentiell übertragen.

Auf diese Weise lässt sich auch bspw. bei gleichbleibender Übertragungsqualität und gleichem Außendurchmesser des Sternviererkabels eine größere Leiterlänge verwenden.

Bei einer weiteren bevorzugten Ausgestaltung der Erfindung weist das Sternviererkabel mindestens ein erstes Adernpaar mit zwei einander gegenüberliegenden Adern und ein zweites Adernpaar mit zwei einander gegenüberliegenden Adern aufweist, wobei die Adern des ersten Adernpaars einen anderen Durchmesser als die Adern des zweiten Adernpaars aufweisen.

Insbesondere weist das Adernpaar, das zur Übertragung der Bilddaten ausgebildet ist, einen größeren Durchmesser als das andere Adernpaar auf, das zur Übertragung des Taktsignals ausgebildet ist.

Da an die Übertragung der Bilddaten generell höhere Anforderungen bezüglich der Übertragungsqualität gestellt werden, ist es auf diese Weise möglich, eine optimale Übertragungsqualität der Bilddaten bei gleichbleibendem Außendurchmesser des Datenübertragungselements bereitzustellen.

Bei der Erfindung weist das Sternviererkabel mindestens ein erstes Adernpaar mit zwei einander gegenüberliegenden Adern und ein zweites Adernpaar mit zwei einander gegenüberliegenden Adern auf, wobei in den Zwischenräumen zwischen dem ersten Adernpaar und dem zweiten Adernpaar mindestens eine zusätzliche Ader angeordnet ist.

Dabei ist der Begriff "zusätzliche Ader" nicht dahingehend einschränkend zu verstehen, dass die zusätzliche Ader zwingend dazu geeignet sein muss, ein elektrisches Signal übertragen zu können. Es kann sich bei der zusätzlichen Ader insbesondere auch um einen Lichtleiter handeln.

Eine zusätzliche Ader wird bevorzugt im Zentrum des Datenübertragungselements angeordnet und insbesondere als Masseleiter ausgebildet. Alternativ oder zusätzlich wird bevorzugt eine weitere zusätzliche Ader bzw. werden weitere zusätzliche Adern derart angeordnet, dass ein Zentrum einer solchen weiteren zusätzlichen Ader in einem größeren radialen Abstand zum Zentrum des Datenübertragungselements angeordnet ist, als der radiale Abstand der Zentren der Adern der Adernpaare zum Zentrum des Datenübertragungselements beträgt.

Bevorzugt ist der größte radiale Abstand einer solchen weiteren zusätzlichen Ader - bei Betrachtung des Querschnitts der Ader - zum Zentrum des Datenübertragungselements kleiner oder gleich dem größten radialen Abstand der Adern der Adernpaare - bei Betrachtung des Querschnitts der Adern. Dadurch bleibt trotz der Aufnahme von zusätzlichen Adern der äußere Durchmesser des Datenübertragungselements unverändert.

Bei einer weiteren bevorzugten Ausgestaltung der Erfindung ist die mindestens eine zusätzliche Ader zur Spannungsversorgung des mindestens einen optischen Sensors oder zur Steuerung des mindestens einen optischen Sensors oder als Lichtleiter ausgebildet.

Auf diese Weise können in das Datenübertragungselement bei gleichbleibendem Außendurchmesser zusätzliche Leitungen bspw. zur Spannungsversorgung oder zur Steuerung des Bildsensors in das Datenübertragungselement integriert werden.

Hierdurch wird der Vorteil erreicht, dass neben der Übertragung der Daten von und zu dem mindestens einen optischen Sensor auch eine Lichtübertragung von dem proximalen Ende, bspw. von der Versorgungseinheit, zu dem distalen Ende möglich ist, ohne den Außendurchmesser des Datenübertragungselements zu erhöhen. Wenn als proximalseitige Lichtquelle so genannte Weißlichtlaser (RGB) eingesetzt werden, ist ein Einsatz von dünnen Lichtleitfasern mit einem Außendurchmesser von etwa 0,4 mm bis ungefähr 0,125 mm möglich.

Bei einer weiteren bevorzugten Ausgestaltung der Erfindung ist ein erstes Signal zur Übertragung von Bilddaten und ein zweites Signal zur Übertragung eines Taktsignals ausgebildet. Insbesondere werden das erste und das zweite Signal differentiell übertragen.

Bei einer weiteren bevorzugten Ausgestaltung der Erfindung weist das Instrument einen ersten und einen zweiten optischen Sensor auf, wobei ein erstes differentielles Signal zur Übertragung von Bilddaten und ein zweites differentielles Signal zur Übertragung eines Taktsignals des ersten optischen Sensors ausgebildet ist, und wobei ein drittes differentielles Signal zur Übertragung von Bilddaten und ein viertes differentielles Signal zur Übertragung eines Taktsignals des zweiten optischen Sensors ausgebildet ist.

Bei einer weiteren bevorzugten Ausgestaltung der Erfindung erfolgt die Übertragung der Bilddaten mittels des MIPI CSI-2-Standards.

Die Übertragung nach diesem Standard ermöglicht den Einsatz standardisierter optischer Sensoren und entsprechender Empfangsmodule aus dem Endverbrauchermarkt im Telekommunikationsbereich und eine entsprechend kostengünstige Herstellung der endoskopisch Instrumente.

Insbesondere zusammen mit der Übertragung von drei differentiellen Signalen ergibt sich dann auch die Möglichkeit, den MIPI CSI-2 "two lane" Standard zu nutzen. Bei Verwendung von vier differentiellen Signalen können zwei optische Sensoren jeweils mit dem MIPI CSI-2 "one lane" Standard genutzt werden.

Bei einer weiteren bevorzugten Ausgestaltung der Erfindung ist an dem distalen Ende eine Treibereinheit mit Präemphase zur Vorverzerrung der Bilddaten des mindestens einen optischen Sensors angeordnet.

Eine distalseitige Verstärkung und Vorverzerrung erlaubt bei einem gegebenen Außendurchmesser des Datenübertragungselements eine weitere Vergrößerung der Kabellänge bei gleichbleibender Übertragungsqualität.

Bei einer weiteren bevorzugten Ausgestaltung der Erfindung ist an dem proximalen Ende eine Entzerrereinheit zur Entzerrung der Bilddaten des mindestens einen optischen Sensors angeordnet. Die Entzerrereinheit kann alternativ oder kumulativ zu der Treibereinheit mit Präemphase angeordnet sein.

Bei gegebenem Außendurchmesser des Datenübertragungselements kann so bei gleichbleibender Übertragungsqualität eine noch größere Kabellänge verwendet werden. Insbesondere wird die Entzerrereinheit vorzugsweise als ein Teil der Versorgungseinheit ausgebildet.

Bei einer weiteren bevorzugten Ausgestaltung der Erfindung weist das Sternviererkabel einen Durchmesser von weniger als 5 mm, bevorzugt weniger als 4 mm, besonders bevorzugt weniger als 3 mm auf und gleichzeitig eine Länge von mindestens 0,5 m, bevorzugt mindestens 1,0 m, besonders bevorzugt 1,5 m auf. Dabei weist das Datenübertragungselement bevorzugt gleichzeitig eine Übertragungsrate von mindestens 400 MBit/s, bevorzugt mindestens 800 MBit/s, insbesondere von 960 MBit/s auf.

Bei einer weiteren bevorzugten Ausgestaltung der Erfindung ist dem mindestens einen optischen Sensor mindestens eine optische Einheit zugeordnet, die distalseitig vor dem mindestens einen optischen Sensor angeordnet ist. Eine vorteilhafte Weiterbildung der Erfindung sieht vor, dass die optische Einheit auf ihrer einen Seite eine Kupplungseinheit aufweist und die es ermöglicht, dass die optische Einheit lösbar distal vor dem mindestens einen optischen Sensor angebracht und damit vorübergehend im Eingriff festgehalten werden kann.

Bei der optischen Einheit oder dem lösbaren Teil der optischen Einheit kann es sich bspw. um einen Strahlteiler, einen Filter, mindestens eine Linse, insbesondere eine Stablinse, ein Prisma oder ein Objektiv oder auch um ein endoskopisches Instrument handeln. Ist mehr als ein optischer Sensor angeordnet, kann eine optische Einheit für jeden der optischen Sensoren angeordnet sein, es kann die optische Einheit aber auch für mehrere oder alle optischen Sensoren gemeinsam vorgesehen sein.

Weitere Vorteile und Ausgestaltungen ergeben sich aus der Zeichnung und der folgenden Figurenbeschreibung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Perspektivansicht einer Ausführungsform eines endoskopischen Instruments;
- Fig. 2: eine Querschnittsansicht einer ersten Ausführungsform eines Datenübertragungselements, die nicht Teil der Erfindung ist;
- Fig. 3: eine Querschnittsansicht einer zweiten Ausführungsform eines Datenübertragungselements;
- Fig. 4: eine beispielhafte bemaßte Ausführungsform des Datenübertragungselements in Fig. 3;
- Fig. 5: eine Verschaltungsanordnung einer Ausführungsform eines endoskopischen Instruments;
- Fig. 6: eine weitere Ausführungsform einer Verschaltungsanordnung eines endoskopischen Instruments;
- Fig. 7: noch eine weitere Ausführungsform einer Verschaltungsanordnung eines endoskopischen Instruments;
- Fig. 8: eine Querschnittsansicht noch einer weiteren Ausführungsform eine Datenübertragungselements;
- Fig. 9: den zeitlichen Verlauf eines beispielhaften Taktsignals;
- Fig. 10: ein beispielhaftes Bildsignal bei einem Datenübertragungselement;
- Fig. 11: ein beispielhaftes Taktsignal bei Verwendung eines Datenübertragungselement, das gemäß Fig. 8 ausgebildet ist;
- Fig. 12: ein beispielhaftes Bildsignal bei Verwendung eines Datenübertragungselement, das gemäß Fig. 8 ausgebildet ist;
- Fig. 13: eine schematische Ansicht noch einer weiteren Ausführungsform eines endoskopischen Instruments;
- Fig. 14: eine schematische Ansicht noch einer weiteren Ausführungsform eines endoskopisch Instruments;
- Fig. 15: ein Bildsignal bei Verwendung der Ausführungsform in Fig. 14 bei Umgehung einer Entzerrereinheit; und
- Fig. 16: ein Bildsignal bei Verwendung der Ausführungsform in Fig. 14 bei Verwendung einer Entzerrereinheit.

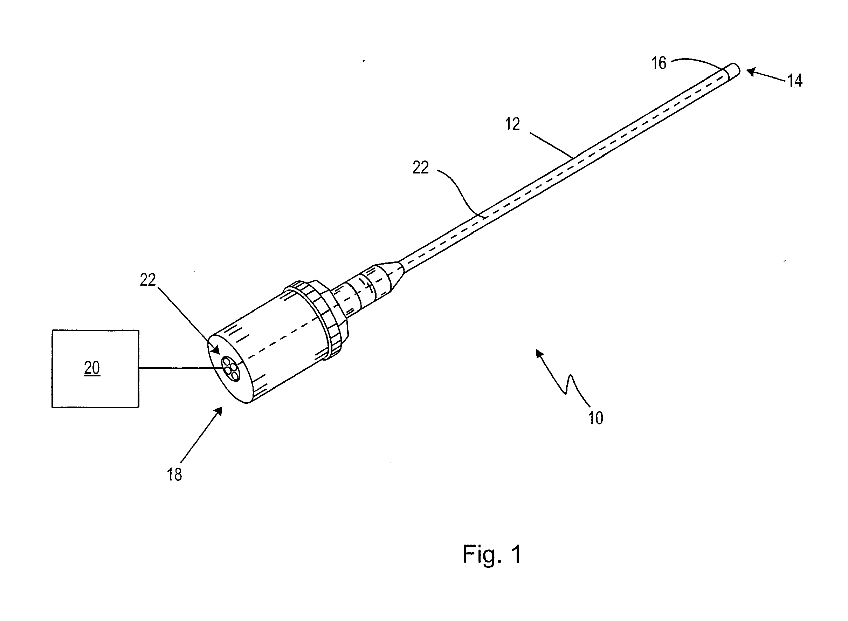

Fig. 1 zeigt eine Ausführungsform eines endoskopischen Instruments 10. Das endoskopische Instrument 10, das in der dargestellten Ausführungsform als Endoskop ausgebildet ist, weist einen Schaft 12 auf, der wiederum ein distales Ende 14, an dem ein optischer Sensor 16 angeordnet ist, und ein proximales Ende 18 zum Anschluss an eine Versorgungseinheit 20 aufweist. Zwischen dem optischen Sensor 16 und dem proximalen Ende 18 ist ein Datenübertragungselement angeordnet. Das Datenübertragungselement ist dafür ausgebildet, mindestens zwei Signale differentiell zu übertragen. Das Datenübertragungselement ist als Sternviererkabel 22 ausgebildet. Des Weiteren ist in einer Weiterbildung vorgesehen, dass das Sternviererkabel 22 über das proximale Ende 18 hinaus bis zu der Versorgungseinheit 20 verläuft. Außerdem kann dem optischen Sensor 16 mindestens eine optische Einheit (nicht dargestellt) zugeordnet sein, die distalseitig bzw. strahlseitig vor dem mindestens einen optischen Sensor 16 angeordnet ist. Der optische Sensor 16 muss also nicht zwingend den distalseitigen Abschluss des endoskopischen Instruments 10 bilden.

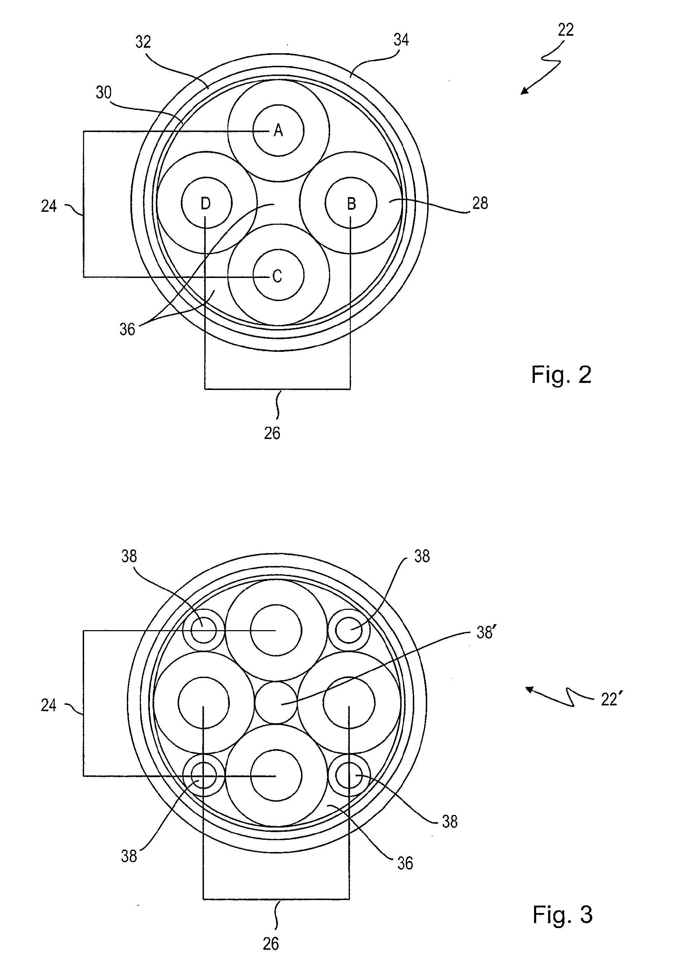

Fig. 2 zeigt einen schematischen Querschnitt durch eine Ausführungsform des Datenübertragungselements, das als Sternviererkabel 22 ausgebildet ist und nicht Teil der Erfindung ist.

Das Sternviererkabel 22 weist ein erstes Adernpaar 24 und ein zweites Adernpaar 26 auf. Das erste Adernpaar 24 weist die Adern A und C auf, das zweite Adernpaar 26 weist die Adern B und D auf. Die Adern A und C liegen einander gegenüber, ebenso wie die Adern B und D einander gegenüberliegen.

Die Adern A bis D sind jeweils von einer Isolation 28 umgeben und sind in einer, in der dargestellten Ausführungsform dreischichtigen, Ummantelung angeordnet. Zunächst sind beide Adernpaare 24, 26 von einer Folie 30 umgeben. Um die Folie 30 herum ist ein Schirm 32 angeordnet. Um den Schirm 32 herum ist ein Mantel 34 des Sternviererkabels 22 angeordnet.

Zwischen den einzelnen Adern A bis D weist das Sternviererkabel 22 Zwischenräume 36 auf.

Fig. 3 zeigt eine zweite bevorzugte Ausführungsform des Sternviererkabels 22'. Diese Ausführungsform weist ebenfalls zwei Adernpaare 24, 26 auf. In den Zwischenräumen 36 sind jedoch zusätzliche Adern 38, 38' angeordnet, die bspw. Steuersignale übertragen können oder der Stromversorgung des optischen Sensors 16 dienen. Bei den zusätzlichen Adern 38, 38' kann es sich aber auch um Lichtleiter handeln. Es wird deutlich, dass diese zusätzlichen Adern 38, 38' in das Sternviererkabel 22' aufgenommen sind, ohne dass sich dadurch ein Außendurchmesser des Sternviererkabels 22' gegenüber der in Fig. 2 gezeigten Ausführungsform 22 ändert. Auch wird es so möglich, einen Masserückleiter 38' als zusätzliche Ader 38 ausgebildet in einem Zwischenraum 36 zu verlegen.

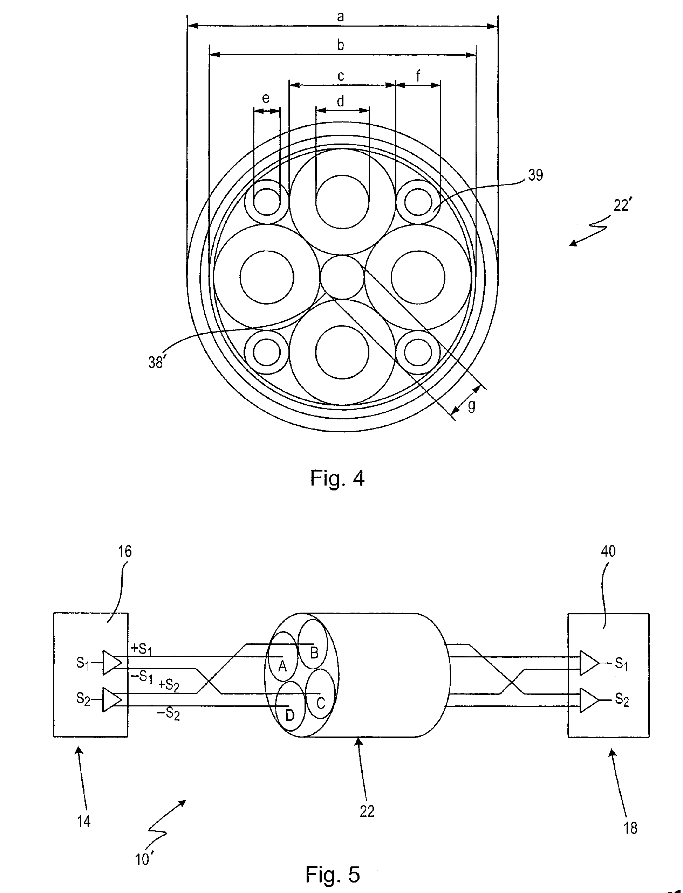

Fig. 4 zeigt eine bemaßte Ansicht des Sternviererkabels 22' in Fig. 3.

Ein Außendurchmesser a beträgt vorzugsweise weniger als 5 mm, bevorzugter weniger als 4 mm, und noch bevorzugter weniger als 3 mm. In der dargestellten Ausführungsform des Sternviererkabels 22' beträgt der Außendurchmesser a 2,5 mm. Der Durchmesser b, der den Außendurchmesser der Folie 30 wiedergibt, beträgt 2,1 mm.

Der Außendurchmesser der Isolation jeder Ader A bis D ist mit c bezeichnet und beträgt in der dargestellten Ausführungsform 0,8 mm. Der Außendurchmesser jeder Ader A bis D beträgt 0,4 mm und ist mit d bezeichnet.

Der Außendurchmesser jeder zusätzlichen Ader 38 ist mit e bezeichnet und beträgt hier 0,2 mm. Auch jede zusätzliche Ader 38 weist bevorzugt jeweils eine eigene Isolation 39 auf, deren Außendurchmesser mit f bezeichnet ist und in der dargestellten Ausführungsform 0,3 mm beträgt.

Auch ein Außendurchmesser einer zentral angeordneten zusätzlichen Ader 38', die im dargestellten Beispiel als Masserückleiter ausgebildet ist, beträgt 0,3 mm, dieser Außendurchmesser ist mit g bezeichnet.

Mittels des dargestellten Sternviererkabels 22' lassen sich besonders gute elektrische Eigenschaften für einen Einsatz in dem endoskopischen Instrument 10 erzielen. So beträgt ein differentieller Wellenwiderstand eines Adernpaars vorzugsweise 100 Ω. Die Dämpfung bei 400 MHz beträgt weniger als 1,0 dB/m, insbesondere 0,8 dB/m oder weniger.

Der Gleichstrom-Leiterwiderstand beträgt in den Adern A bis D weniger als 0,5 Ω/m, insbesondere 0,2 Ω/m und weniger, in den zusätzlichen Adern 38 weniger als 1,0 Ω/m, insbesondere 0,58 Ω/m und weniger. In der als Masserückleiter ausgebildeten zusätzlichen Ader 38' beträgt der Gleichstrom-Leiterwiderstand weniger als 0,5 Ω/m, insbesondere 0,39 Ω/m und weniger.

Für die jeweilige Isolation 28, 39 ist ein Material gewählt, so dass ein Isolationswiderstand von 1500 MΩ km vorliegt. Die Prüfspannung beträgt jeweils 500 Volt.

Fig. 5 zeigt schematisch eine Verschaltungsanordnung einer Ausführungsform 10' des endoskopischen Instruments.

Die Verschaltungsanordnung in Fig. 5 ist dazu ausgebildet, zwischen dem optischen Sensor 16 und dem proximalen Ende 18 zwei Signale s1 und s2 differentiell zu übertragen.

An dem proximalen Ende 18 weist das endoskopische Instrument 10' eine Kontrolleinheit mit Verstärker 40 auf, die die Signale s1 und s2 empfängt und an die Versorgungseinheit 20 (nicht dargestellt) übermittelt.

Das differentielle Signal +s1/-s1 wird über das erste Adernpaar 24 übertragen, indem z.B. ein Signalteil +s1 an die Ader A und ein Signalteil -s1 an die Ader C geleitet wird.

Das zweite differentielle Signal +s2/-s2 wird über das zweite Adernpaar 26 übertragen, indem z.B. ein Signalteil +s2 an die Ader B und ein Signalteil -s2 an die Ader D geleitet wird. Aus Symmetriegründen heben sich die durch die Leiteradern A und C verursachten entgegengesetzten Felder an den Positionen der Adern B und D auf und umgekehrt. Deswegen beeinflussen sich die beiden Signale s1 und s2 nicht, das heißt, es findet kein Übersprechen zwischen dem ersten Adernpaar 24 und dem zweiten Adernpaar 26 statt.

Die dargestellte Verschaltungsanordnung ist bspw. zur Übertragung des MIPI CSI-2 "one lane" Standard geeignet.

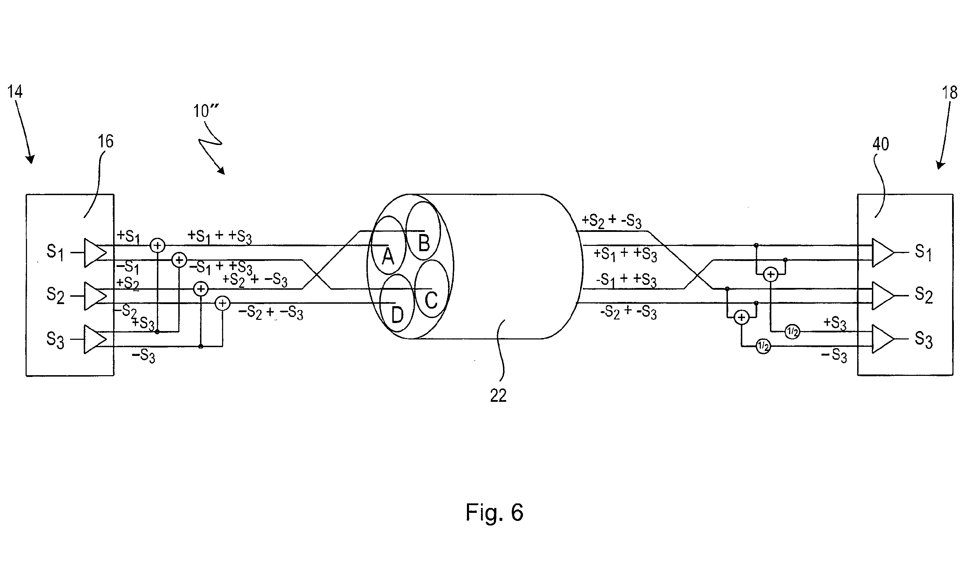

Fig. 6 zeigt eine Ausführungsform 10" des endoskopischen Instruments mit einer Verschaltungsanordnung zwischen dem optischen Sensor 16 und dem proximalen Ende 18, die dazu geeignet ist, mittels des Sternviererkabels 22 drei Signale s1, s2, s3 differentiell zu übertragen. Die Übertragung des ersten differentiellen Signals +s1/-s1 und des zweiten differentiellen Signals +s2/-s2 erfolgt mittels einer Verschaltung, die der in Fig. 5 gezeigten entspricht.

Ein drittes differentielles Signal +s3/-s3 wird zusätzlich über die Adernpaare 24 und 26 übertragen. Dabei wird z.B. ein Signalteil +s3 auf die Signale der Adern A und C addiert, während ein Signalteil -s3 auf die Signale der Adern B und D addiert wird. Aus Symmetriegründen sind die durch die Signalteile +s3 und - s3 verursachten Felder an den Positionen der Leiter A und C gleich stark, ebenso wie an den Positionen der Leiter B und D. Daher werden die differentiellen Signale des ersten Adernpaars 24 und des zweiten Adernpaars 26 nicht von dem dritten differentiellen Signal +s3/-s3 beeinflusst.

Die Entschlüsselung des dritten differentiellen Signals +s3/-s3 erfolgt, indem an dem proximalen Ende 18 die Signale der Adern A und C erneut addiert werden. Die Signalteile +s1 und -s1 heben sich bei der Addition gegenseitig auf, so dass man dann einen Signalteil von zweimal +s3 erhält. Durch Halbierung des Signals erlangt man so den Signalteil +s3. Entsprechend gelangt man durch Addition der Signale der Adern B und D und anschließendes Halbieren des Signals zu dem Signalteil -s3, da sich die Signalteile +s2 und -s2 beim Addieren gegenseitig aufheben.

Diese Verschaltungsanordnung eignet sich bspw. für den MIPI CSI-2 "two lane" Standard. Dabei wird bspw. ein Bilddatensignal auf zwei Kanäle bzw. zwei Signale aufgeteilt. Durch die bei gleicher Bildauflösung geringere Übertragungsbandbreite pro Signal können so längere Distanzen bei gleich bleibender Signalqualität überbrückt werden.

Des Weiteren wird es bspw. auch möglich, auf diese Weise zwei verschiedene Bilddatensignale (s1 und s2) zu übertragen und gleichzeitig von dem proximalen Ende 18 aus ein Taktsignal (s3) an das distale Ende 14 zu übertragen.

Auf diese Weise können an dem distalen Ende 14 vorzugsweise auch zwei optische Sensoren in einer Stereoanordnung angeordnet sein.

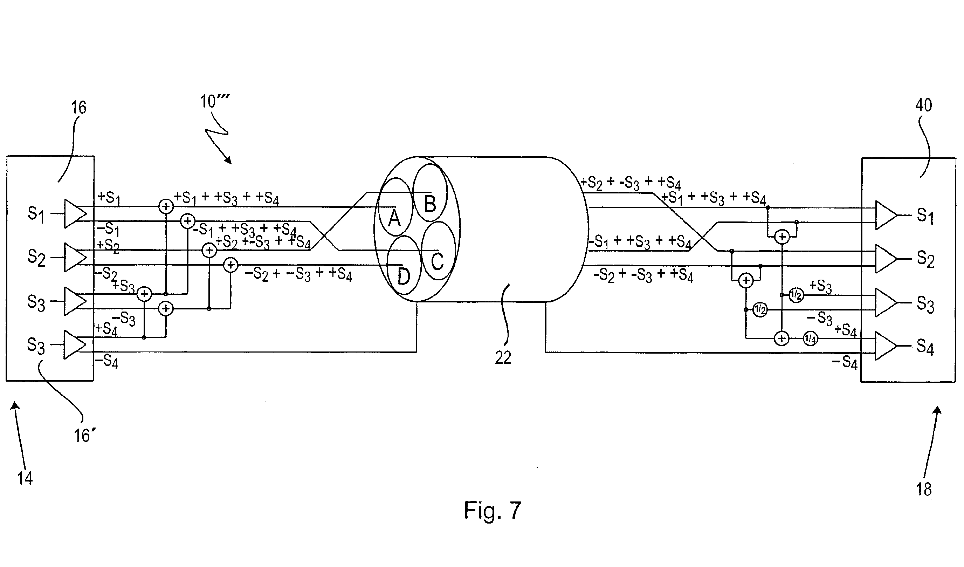

Fig. 7 zeigt eine weitere Ausführungsform 10" des endoskopischen Instruments mit einer Verschaltungsanordnung, die dazu geeignet ist, vier Signale s1 bis s4 differentiell zu übertragen.

Die Verschaltungsanordnung zur Übertragung der ersten drei Signale s1 bis s3 entspricht dabei der in Fig. 6 gezeigten.

Ein viertes differentielles Signal +s4/-s4 wird übertragen, indem z.B. ein Signalteil +s4 auf alle vier Adern A bis D addiert wird, während ein Signalteil -s4 auf die Gesamtabschirmung des Sternviererkabels 22' geschaltet wird. Aufgrund der differentiellen Übertragung werden die anderen drei Signale s1 bis s3 durch dieses vierte Signal s4 nicht beeinflusst.

Die Ermittlung des vierten Signals s4 in der Kontrolleinheit 40 an dem proximalen Ende 18 erfolgt dann bevorzugt, indem die Signalteile der Adern A bis D aufaddiert werden und das sich daraus ergebende Signal dann geviertelt wird. Die Signalteile +s1, -s1, +s2, -s2, +s3, -s3 heben sich jeweils paarweise gegenseitig auf, so dass man ein Summensignal von viermal +s4 erhält, das geviertelt der Signalteil +s4 ergibt. Der Signalteil -s4 wird direkt aus der Gesamtabschirmung abgegriffen, so dass die Kontrolleinheit 40 das Signal s4 ermitteln kann.

In einer nicht dargestellten bevorzugten Ausführungsform werden drei Signale s1, s2 und s4 differentiell übertragen. Das heißt, ein drittes Signal muss nicht zwangsläufig über die in Fig. 6 dargestellte Verschaltungsanordnung übertragen werden, sondern kann auch durch die in Fig. 7 für das Signal s4 dargestellte Verschaltung übertragen werden. Die in Fig. 7 für das Signal s4 dargestellte Verschaltungsanordnung ist somit auch ohne Übertragung eines Signals s3 möglich. Es ist lediglich ein Signalteil +s4 auf alle vier Adern A bis D und der Signalteil -s4 auf die Gesamtabschirmung zu schalten.

Mittels der vier Signale s1 bis s4 lassen sich zwei optische Sensoren 16 und 16' gleichzeitig und unabhängig voneinander jeweils in dem MIPI CSI-2 one lane Standard betreiben. Diese Anordnung ist also z.B. für Stereoanordnungen mit zwei distalseitigen optischen Sensoren 16, 16' geeignet. Darüber hinaus sind Anordnungen mit zwei optischen Sensoren für unterschiedliche Blickrichtungen oder unterschiedliche spektrale Empfindlichkeiten, etwa in Fluoreszenzanwendungen, realisierbar.

Es versteht sich, dass es sich bei den optischen Sensoren 16 und 16' sowohl um Einzelbildsensoren handeln kann, wie sie bspw. zur Aufnahme von Fotos verwendet werden, aber auch kontinuierlich aufzeichnende Sensoren handeln kann, die für Videoanwendungen geeignet sind.

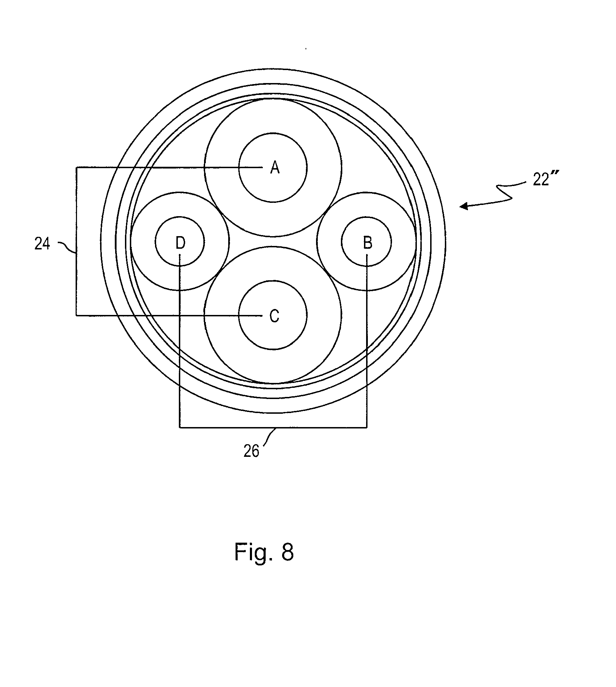

Fig. 8 zeigt eine weitere Ausführungsform des Sternviererkabels 22". Ein erstes differentielles Signal s1, das mittels des ersten Adernpaars 24 übertragen wird, ist zur Übertragung von Bilddaten ausgebildet und ein zweites differentielles Signal s2, das mittels des zweiten Adernpaars 26 übertragen wird, ist zur Übertragung eines Taktsignals ausgebildet. Beispielhaft sind lediglich zwei Signale dargestellt, die folgenden Ausführungsformen können auch zusammen mit drei oder vier Signalen verwendet werden.

Der Außendurchmesser des ersten Adernpaars 24 weist jedoch einen anderen Durchmesser als das zweite Adernpaar 26 auf. Insbesondere sind die Durchmesser der Adern A und C des ersten Adernpaars 24 größer als die Durchmesser der Adern B und D des zweiten Adernpaars 26.

Diese Anordnung empfiehlt sich insbesondere, wenn ein periodisch zwischen zwei Zuständen wechselndes Signal, wie z.B. das Taktsignal, und ein nichtperiodisches Signal, wie z.B. das Bilddatensignal, übertragen werden. Für das periodische Signal kann eine größere Dämpfung akzeptiert werden, da hier kein dämpfungsverursachter deterministischer Jitter (Schwankung) auftritt. Deshalb kann für dieses Signal ein geringerer Leiterdurchmesser gewählt werden. Die Fehlerquelle des deterministischen Jitters ist dem Fachmann bekannt. Es erlaubt jedoch gleichzeitig bei gleichem Außendurchmesser einen größeren Durchmesser für das andere Adernpaar, in dem in Fig. 8 dargestellten Beispiel des ersten Adernpaars 24. Auf diese Weise wird für das nicht-periodische Signal eine geringere Dämpfung erzielt.

Insbesondere bei einer Datenübertragung mittels des MIPI CSI-2-Standards kann durch die Unterscheidung zwischen Taktsignal und Datensignal eine größere Dämpfung des Taktsignals zugunsten einer geringeren Dämpfung des Bilddatensignals in Kauf genommen werden.

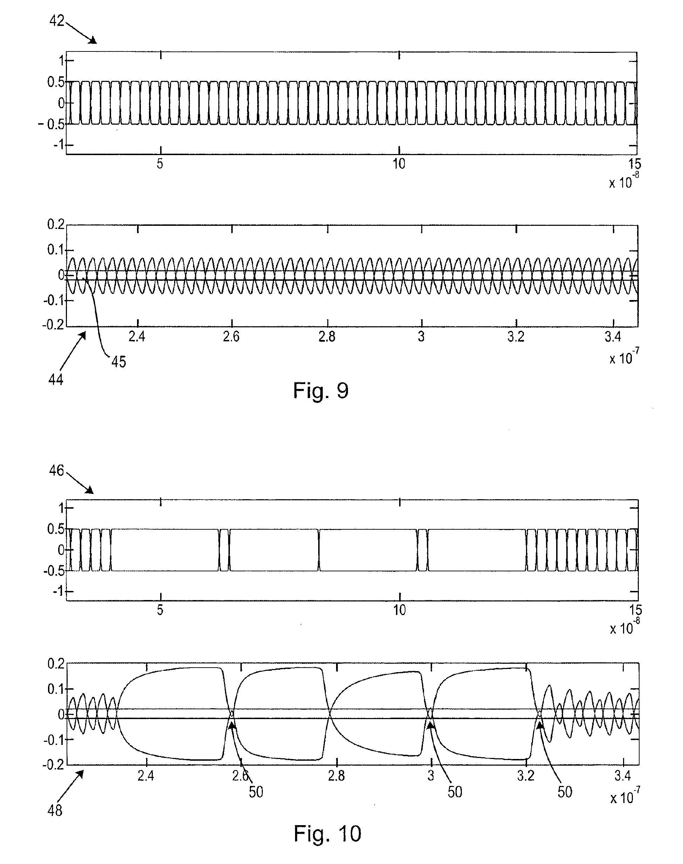

In Fig. 9 ist ein differentielles Taktsignal bei Übertragung mittels eines Sternviererkabels 22 gezeigt, das als Sternviererkabel ausgebildet ist und bei dem das erste Adernpaar 24 und das zweite Adernpaar 26 gleiche Durchmesser aufweisen. Ein Sendesignal ist mit dem Bezugszeichen 42 und ein Empfangssignal mit dem Bezugszeichen 44 gekennzeichnet. In dem Empfangssignal 44 ist eine Entscheiderschwelle 45 markiert, die überschritten werden muss, damit ein Bit von einem Empfänger einwandfrei detektiert werden kann.

In Fig. 10 ist ein gleichzeitig mit dem Taktsignal in Fig. 9 übertragendes Bilddatensignal gezeigt. Das senderseitige Bilddatensignal ist mit dem Bezugszeichen 46, das empfangsseitige Bilddatensignal mit dem Bezugszeichen 48 gekennzeichnet. Wie zu erkennen ist, können nicht alle Bits fehlerfrei identifiziert werden.

Mit Pfeilen 50 sind Signalstellen gekennzeichnet, bei denen die Amplitude nicht über die Entscheiderschwelle hinausgeht, so dass das entsprechende Bit nicht fehlerfrei detektiert werden kann. Problematisch sind stets lange Folgen von 0-Bits (bzw. 1-Bits), die von einzelnen 1-Bits (bzw. 0-Bits) unterbrochen werden. Die Augenöffnungen des Empfangssignals 48 sind an den fehlerhaften Stellen 50 offensichtlich deutlich geringer als sie bei dem empfangsseitigen Taktsignal 44 in Fig. 9 vorhanden sind.

Für diese Fälle eignet sich insbesondere der in Fig. 8 dargestellte Aufbau des Sternviererkabels 22".

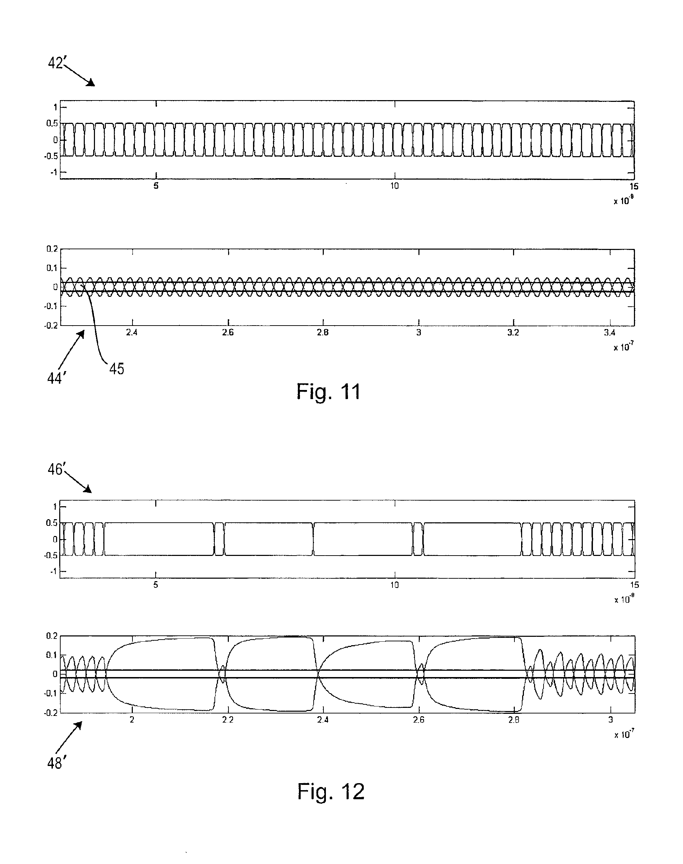

Die Figuren 11 und 12 zeigen entsprechend ein senderseitiges Taktsignal 42' und ein senderseitiges Bilddatensignal 46' sowie ein empfängerseitiges Taktsignal 44' und ein empfängerseitiges Bilddatensignal 48'.

Ein Außendurchmesser des Datenübertragungselements ist dabei derselbe wie bei den in Figuren 9 und 10 übertragenen Signalen. Es ist zu erkennen, dass das empfängerseitige Taktsignal 44' zwar eine geringere Amplitude als das in Fig. 9 aufweist, die Entscheiderschwelle 45 wird aber immer noch stets überschritten.

Auch das empfängerseitige Datensignal 48' überschreitet nun stets die Entscheiderschwelle 45. Dies wird erreicht durch die geringere Dämpfung des ersten Adernpaars 24 mit größerem Durchmesser, was in Fig. 8 dargestellt ist. Die Augenöffnungen bei dem empfängerseitigen Taktsignal 44' und dem empfängerseitigen Bilddatensignal 48' sind nun durchweg in etwa gleich groß.

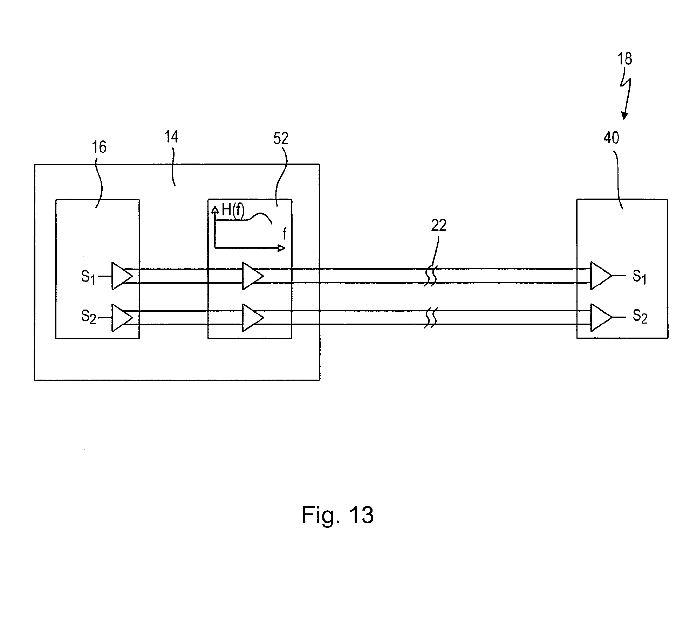

Fig. 13 zeigt eine weitere mögliche Ausgestaltung des endoskopischen Instruments 10, die alternativ oder kumulativ zu den voranstehend beschriebenen Ausgestaltungen des Sternviererkabels 22 eingesetzt werden kann, um die Übertragungsqualität der Signale s1 bis s4 weiter zu erhöhen.

Hierzu ist an dem distalen Ende 14 eine Treibereinheit 52 angeordnet, an der eine senderseitige Vorverzerrung mit Präemphase vorgenommen wird.

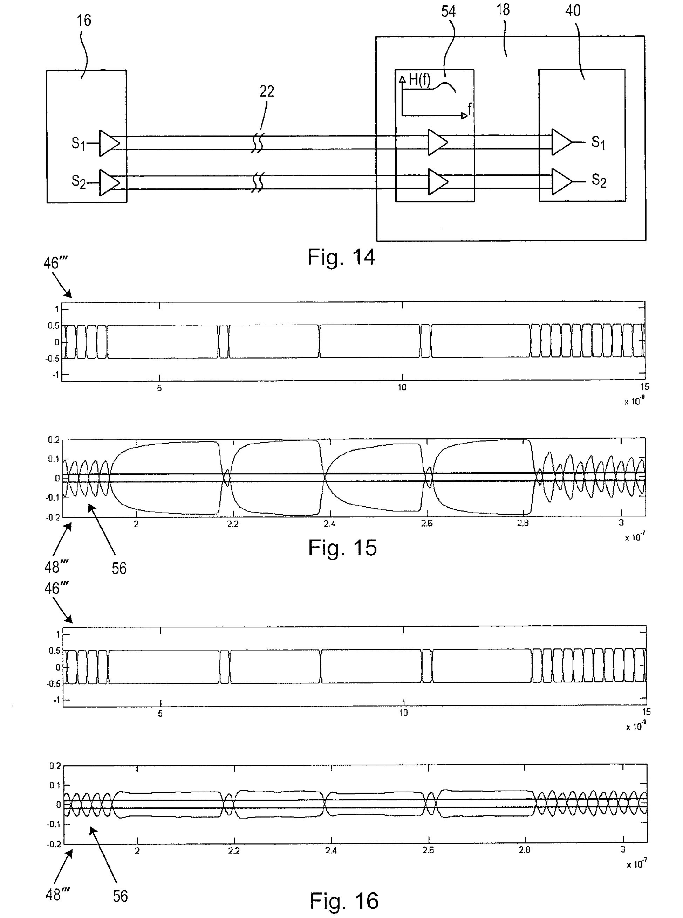

In Fig. 14 ist dargestellt, dass alternativ auch eine empfängerseitige Entzerrung mittels einer Entzerrereinheit 54 an dem proximalen Ende 18 durchgeführt werden kann. Die empfängerseitige Entzerrung kann grundsätzlich auch kumulativ mit der Vorverzerrung durchgeführt werden.

Durch die senderseitige Vorverzerrung oder die empfängerseitige Entzerrung erfolgt eine Anhebung der hochfrequenten Spektralanteile, um die Dämpfungseigenschaften des Sternviererkabels 22 (zunehmende Dämpfung mit zunehmender Frequenz) auszugleichen. Auf diese Weise kann die maximal mögliche Länge des Sternviererkabels 22 bei gleichem Außendurchmesser signifikant erhöht werden.

Die Auswirkungen auf die empfängerseitigen Signale sind in den Figuren 15 und 16 dargestellt.

Fig. 15 zeigt den zeitlichen Verlauf eines senderseitigen Bilddatensignals 46" und eines empfängerseitigen Bilddatensignals 48" ohne Entzerrung.

Fig. 16 zeigt den zeitlichen Verlauf eines senderseitigen Bilddatensignals 46"' und eines empfängerseitigen Bilddatensignals 48"' bei gleichem Aufbau, wobei jedoch eine Entzerrereinheit 54 verwendet wird.

Eine Maximalamplitude des empfängerseitigen Signals 48"' ist gegenüber einer Maximalamplitude des empfängerseitigen Signals 48" deutlich verringert. Dafür ist jedoch die Maximalamplitude von Einzelbits 56 deutlich erhöht. Dadurch vergrößern sich auch die Augenöffnungen dieser Einzelbits 56, so dass eine sicherere Detektion der Einzelbits 56 möglich wird.

Die Übertragungsqualität kann so noch zusätzlich erhöht werden.

## Patentansprüche

1. Endoskopisches Instrument (10) mit einem Schaft (12), der ein distales Ende (14), an dem mindestens ein optischer Sensor (16, 16') angeordnet ist, und ein proximales Ende (18) zum Anschluss an eine Versorgungseinheit (20) aufweist, und mit einem Datenübertragungselement, das zwischen dem mindestens einen optischen Sensor (16, 16') und dem proximalen Ende (18) angeordnet ist und dafür ausgebildet ist, mindestens zwei Signale (s₁, s₂) differentiell zu übertragen, **dadurch gekennzeichnet, dass** das Datenübertragungselement als Sternviererkabel (22) ausgebildet ist und das Sternviererkabel (22) mindestens ein erstes Adernpaar (24) mit zwei einander gegenüberliegende Adern (A, C) und ein zweites Adernpaar (26) mit zwei einander gegenüberliegenden Adern (B, D) aufweist, wobei in Zwischenräumen (36) zwischen dem ersten Adernpaar (24) und dem zweiten Adernpaar (26) mindestens eine zusätzliche Ader (38) angeordnet ist.

2. Endoskopisches Instrument nach Anspruch 1, **dadurch gekennzeichnet, dass** das der mindestens eine optische Sensor (16, 16') mit dem Sternviererkabel (22) derart verbunden ist, dass mindestens drei Signale (s₁, s₂, s₃) differentiell übertragen werden können.

3. Endoskopisches Instrument nach Anspruch 1 oder 2, **dadurch gekennzeichnet, dass** der mindestens eine optische Sensor (16, 16') mit dem Sternviererkabel (22) derart verbunden ist, dass vier Signale (s₁, s₂, s₃, s₄) differentiell übertragen werden können.

4. Endoskopisches Instrument nach einem der Ansprüche 1 bis 3, **dadurch gekennzeichnet, dass** das Sternviererkabel (22) mindestens ein erstes Adernpaar (24) mit zwei einander gegenüberliegenden Adern (A, C) und ein zweites Adernpaar (26) mit zwei einander gegenüberliegenden Adern (B, D) aufweist, wobei die Adern (A, C) des ersten Adernpaars (24) einen anderen Durchmesser als die Adern (B, D) des zweiten Adernpaars (26) aufweisen.

5. Endoskopisches Instrument nach einem der vorhergehenden Ansprüche, **dadurch gekennzeichnet, dass** die mindestens eine zusätzliche Ader (38) zur Spannungsversorgung des mindestens einen optischen Sensors (16) ausgebildet ist oder zur Steuerung des mindestens einen optischen Sensors (16) ausgebildet ist oder als Lichtleiter ausgebildet ist.

6. Endoskopisches Instrument nach einem der Ansprüche 1 bis 5, **dadurch gekennzeichnet, dass** ein erstes Signal (s₁) ein Bilddatensignal (46, 48) und ein zweites Signal (s₂) ein Taktsignal (42, 44) ist.

7. Endoskopisches Instrument nach einem der Ansprüche 3 bis 6, **dadurch gekennzeichnet, dass** das Instrument (10) einen ersten (16) und einen zweiten (16') optischen Sensor aufweist, wobei ein erstes Signal (s₁) ein Bilddatensignal (46, 48) und ein zweites Signal (s₂) ein Taktsignal (42, 44) des ersten optischen Sensors (16) ist, und wobei ein drittes Signal (s₃) ein Bilddatensignal (46, 48) und ein viertes Signal (s₄) ein Taktsignal (42, 44) des zweiten optischen Sensors (16') ist.

8. Endoskopisches Instrument nach Anspruch 6 oder 7, **dadurch gekennzeichnet, dass** die Übertragung der Bilddaten (46, 48) mittels des MIPI CSI-2 Standards erfolgt.

9. Endoskopisches Instrument nach einem der Ansprüche 1 bis 8, **dadurch gekennzeichnet, dass** an dem distalen Ende (14) eine Treibereinheit (52) mit Präemphase zur Vorverzerrung der Bilddaten (46, 48) des mindestens einen optischen Sensors (16, 16') angeordnet ist.

10. Endoskopisches Instrument nach einem der Ansprüche 1 bis 9, **dadurch gekennzeichnet, dass** an dem proximalen Ende (18) eine Entzerrereinheit (54) zur Entzerrung der Bilddaten (46, 48) des mindestens einen optischen Sensors (16, 16') angeordnet ist.

11. Endoskopisches Instrument nach einem der Ansprüche 1 bis 10, **dadurch gekennzeichnet, dass** das Sternviererkabel (22) einen Durchmesser (a) von weniger als 5 mm, bevorzugt weniger als 4 mm, bevorzugter weniger als 3mm, eine Länge von mindestens 0,5 m, bevorzugt mindestens 1,0 m, bevorzugter 1,5 m aufweist.

12. Endoskopisches Instrument nach einem der Ansprüche 1 bis 11, **dadurch gekennzeichnet, dass** dem mindestens einen optischen Sensor (16, 16') mindestens eine optische Einheit zugeordnet ist, die distalseitig vor dem mindestens einen optischen Sensor (16, 16') angeordnet ist und die insbesondere mittels einer Kupplungseinheit zumindest teilweise lösbar ausgebildet ist.

13. Endoskopisches Instrument nach einem der Ansprüche 1 bis 12, **dadurch gekennzeichnet, dass** der optische Sensor (16, 16') eine digitale Schnittstelle nach dem MIPI CSI-2 Standard aufweist.

## Claims

1. Endoscopic instrument (10) comprising a shaft (12), which comprises a distal end (14) at which at least one optical sensor (16, 16') is arranged and comprising a proximal end (18) for connecting a supply unit (20), and comprising a data transfer element, being arranged between the at least one optical sensor (16, 16') and the proximal end (18) and being arranged for differentially transmitting at least two signals (s₁, s₂), **characterized in that** the data transfer element is designed as a star-quad cable (22), wherein the star-quad cable (22) comprises at least a pair of cores (24) comprising two cores (A, C) being arranged opposite with respect to one another and comprising a second pair of cores (26) comprising two cores (B, D) being arranged opposite with respect to one another, wherein in spaces (36) between the first pair of cores (24) and the second pair of cores (26) at least one additional core (38) is arranged.

2. Endoscopic instrument of claim 1 **characterized in that** the at least one optical sensor (16, 16') is connected to the star-quad cable (22) such that at least three signals (s₁, s₂, s₃) can be transferred differentially.

3. Endoscopic instrument of claim 1 or 2, **characterized in that** the at least one optical sensor (16, 16') is connected to the star-quad cable (22) such that four signals (s₁, s₂, s₃, s₄) can be transferred differentially.

4. Endoscopic instrument of any of claims 1 through 3, **characterized in that** the star-quad cable (22) comprises at least a first pair of cores (24) comprising two cores (A, C) being arranged opposite with respect to one another and a second pair of cores (26) comprising two cores (B, D) being arranged opposite with respect to one another, wherein the cores (A, C) of the first pair of cores (24) comprise a different diameter with respect to the cores (B, D) of the second pair of cores (26).

5. Endoscopic instrument of any of the previous claims, **characterized in that** the at least one additional core (38) is designed for voltage supply of the at least one optical sensor (16) or is designed for controlling the at least one optical sensor (16) or is designed as a light guide.

6. Endoscopic instrument of any of claims 1 through 5, **characterized in that** a first signal (s₁) is an image data signal (46, 48) and a second signal (s₂) is a clock signal (42, 44).

7. Endoscopic instrument of any of claims 3 through 6, **characterized in that** the instrument (10) comprises a first (16) and a second (16') optical sensor, wherein a first signal (s₁) is an image data signal (46, 48) and a second signal (s₂) is a clock signal (42, 44) of the first optical sensor (16), and wherein a third signal (s₃) is an image data signal (46, 48) and a fourth signal (s₄) is a clock signal (42, 44) of the second optical sensor (16').

8. Endoscopic instrument of claim 6 or 7, **characterized in that** the transmission of image data (46, 48) is based on the MIPI CSI-2 standard.

9. Endoscopic instrument of any of claims 1 through 8, **characterized in that** a driving unit (52) is arranged at the distal end (14) comprising an accentuation for predistortion of the image data (46, 48) of the at least one optical sensors (16, 16').

10. Endoscopic instrument of any of claims 1 through 9, **characterized in that** an equalizing unit (54) is arranged at the proximal end (18) for equalizing the image data (46, 48) of the at least one optical sensor (16, 16').

11. Endoscopic instrument of any of claims 1 through 10, **characterized in that** the star-quad cable (22) comprises a diameter (a) of less than 5 mm, preferably less than 4 mm, more preferably less than 3 mm, and comprising a length of at least 0.5 m, preferably at least 1.0 m, more preferably 1.5 m.

12. Endoscopic instrument of any of claims 1 through 11, **characterized in that** at least one optical unit is associated with the at least one optical sensor (16, 16'), which is arranged at the distal side of the optical sensor (16, 16') and which, in particular, is designed such that it is at least partially detachable by use of a coupling unit.

13. Endoscopic instrument of any of claims 1 through 12, **characterized in that** the optical sensor (16, 16') comprises a digital interface according to the MIPI CSI-2 standard.

## Revendications

1. Instrument (10) d'endoscopie présentant une tige (12) dotée d'une extrémité distale (14) à laquelle au moins un détecteur optique (16, 16') est disposé et d'une extrémité proximale (18) destinée à être raccordée à une unité d'alimentation (20) et un élément de transfert de données disposé entre le ou les détecteurs optiques (16, 16') et l'extrémité proximale (18) et configuré pour transférer de manière différentielle au moins deux signaux (s₁, s₂), **caractérisé en ce que** l'élément de transfert de données est configuré comme câble (22) à quartes en étoile, **en ce que** le câble (22) à quartes en étoile présente une première paire (24) de fils dotée de deux fils (A, C) mutuellement opposés et une deuxième paire (26) de fils dotée de deux fils (B, D) mutuellement opposés et **en ce qu'**au moins un fil supplémentaire (38) est disposé dans les espaces intermédiaires (36) situés entre la première paire (24) de fils et la deuxième paire (26) de fils.

2. Instrument d'endoscopie selon la revendication 1, **caractérisé en ce que** le ou les détecteurs optiques (16, 16') sont reliés au câble (22) à quartes en étoile de telle sorte qu'au moins trois signaux (s₁, s₂, s₃) puissent être transférés de manière différentielle.

3. Instrument d'endoscopie selon les revendications 1 ou 2, **caractérisé en ce que** le ou les détecteurs optiques (16, 16') sont reliés au câble (22) à quartes en étoile de manière à pouvoir transmettre de manière différentielle quatre signaux (s₁, s₂, s₃, s₄).

4. Instrument d'endoscopie selon l'une des revendications 1 à 3, **caractérisé en ce que** le câble (22) à quartes en étoile présente au moins une première paire (24) de fils dotée de deux fils (A, C) mutuellement opposés et une deuxième paire (26) de fils dotée de deux fils (B, D) mutuellement opposés, les fils (A, C) de la première paire (24) de fils présentant un autre diamètre que les fils (B, D) de la deuxième paire (26) de fils.

5. Instrument d'endoscopie selon l'une des revendications précédentes, **caractérisé en ce que** le ou les fils supplémentaires (38) sont configurés pour alimenter en tension le ou les détecteurs optiques (16), pour commander le ou les détecteurs optiques (16) ou comme conducteurs de lumière.

6. Instrument d'endoscopie selon l'une des revendications 1 à 5, **caractérisé en ce qu'**un premier signal (s₁) est un signal (46, 48) de données d'image et un deuxième signal (s₂) un signal d'horloge (42, 44).

7. Instrument d'endoscopie selon l'une des revendications 3 à 6, **caractérisé en ce que** l'instrument (10) présente un premier détecteur optique (16) et un deuxième détecteur optique (16'), un premier signal (s₁) étant un signal (46, 48) de données d'image et un deuxième signal (s₂) un signal d'horloge (42, 44) du premier détecteur optique (16), un troisième signal (s₃) étant un signal (46, 48) de données d'image et un quatrième signal (s₄) un signal d'horloge (42, 44) du deuxième détecteur optique (16').

8. Instrument d'endoscopie selon les revendications 6 ou 7, **caractérisé en ce que** le transfert des données d'image (46, 48) s'effectue selon la norme MIPI CSI-2.

9. Instrument d'endoscopie selon l'une des revendications 1 à 8, **caractérisé en ce qu'**une unité pilote (52) à phase de préemption pour une pré-distorsion des données d'image (46, 48) du ou des détecteurs optiques (16, 16') est disposée à l'extrémité distale (14).

10. Instrument d'endoscopie selon l'une des revendications 1 à 9, **caractérisé en ce qu'**une unité (54) de dé-distorsion qui assure la dé-distorsion des données d'image (46, 48) du ou des détecteurs optiques (16, 16') est disposée à l'extrémité proximale (18).

11. Instrument d'endoscopie selon l'une des revendications 1 à 10, **caractérisé en ce que** le câble (22) à quartes en étoile présente un diamètre (a) de moins de 5 mm, de préférence de moins de 4 mm, mieux de moins de 3 mm, et une longueur d'au moins 0,5 m, de préférence d'au moins 1,0 m et mieux de 1,5 m.

12. Instrument d'endoscopie selon l'une des revendications 1 à 11, **caractérisé en ce qu'**une unité optique qui est disposée du côté distal en amont du ou des détecteurs optiques (16, 16') et configurée de manière à être au moins en partie libérable au moyen d'une unité d'accouplement est associée au détecteur ou aux détecteurs optiques (16, 16').

13. Instrument d'endoscopie selon l'une des revendications 1 à 12, **caractérisé en ce que** le détecteur optique (16, 16') présente une interface numérique selon la norme MIPI CSI-2.
